# EUROPEAN PATENT SPECIFICATION

(11) **EP 2 559 388 B1**
(45) Date of publication and mention of the grant of the patent: **29.01.2014**
(21) Application number: 12187668.4
(22) Date of filing: 27.04.2005
(51) Int. Cl.: A61B 1/00, A61B 1/018, A61B 1/303, A61B 1/04, A61B 5/00, A61B 17/12, A61B 17/42, A61F 6/22

(54) **Endoscopic delivery of medical devices**
Endoskopische Verabreichung von medizinischen Vorrichtungen
Administration endoscopique de dispositifs médicaux

(30) Priority: 28.04.2004 US 566190 P
(43) Date of publication of application: 20.02.2013
(62) Divisional of application: 05741823.8
(73) Proprietor: Conceptus, Inc., Mountain View, CA 94041 (US)
(72) Inventor: Callister, Jeffery, P., Deephaven, MN 55391 (US); Tremulis, William, S., Redwood City, CA 94065 (US); Kemp, Laura, Los Gatos, CA 95032 (US); Stull Paul, M., Bodega Bay, CA 94923 (US)
(74) Representative: Lloyd, Robin

(56) References cited:
- WO-A-01/13833
- WO-A-94/26175
- US-A- 5 919 202
- US-A- 6 090 063

## Description

### FIELD OF THE INVENTION

This invention relates generally to endoscopes and endoscopic assemblies for delivery of medical devices for therapeutic or diagnostic procedures, particularly for such procedures within a female patient's fallopian tubes.

### BACKGROUND OF THE INVENTION

This invention generally relates to the field of occluding devices, delivery systems for such devices and the method of using such devices and systems in the occlusion of body passageways. The invention is particularly useful for the occluding reproductive lumens such as a female patient's fallopian tubes or a male patient's vas deferens to affect contraception.

Conventional contraceptive strategies generally fall within three categories: physical barriers, drugs and surgery. While each have certain advantages, they also suffer from various drawbacks. Barriers such as condoms and diaphragms are subject to failure due to breakage, displacement and misplacement. Drug strategies, such as the pill and Norplant™, which rely on artificially controlling hormone levels, suffer from known and unknown side-effects from prolonged use. Surgical procedures, such as tubal ligation and vasectomy, are very effective, but involve the costs and attendant risks of surgery, and are frequently not reversible.

Recently, minimally invasive treatments have been proposed which deploy stent-like devices within reproductive lumens for obstructing such lumens as a contraceptive alternative to tubal ligation (See for example U. S. Patent No. 6,432,116). These stent like devices are deployed by delivery catheters having a pushing or holding element disposed within the inner lumen of the delivery catheter proximal to the stent-like device.

Typically, the delivery catheter is advanced through a working lumen of an endoscope such as a hysteroscope, preferably a flexible hysteroscope. Such delivery catheters are described in application Serial No. 10/746,131, filed on December 24, 2003 (publication no. US 2005 085 844 A1). US 5,919,202, from which the preamble of claim 1 is known, describes a known endoscopic instrument with jaws and movable internal needle and method for use thereof.

### SUMMARY OF THE INVENTION

According to the present invention, there is provided an endoscope comprising:
an elongate shaft;
a working channel extending through at least a portion of the elongated shaft;
a loading port in a proximal portion of the elongated shaft for loading an elongated delivery catheter within the working channel; and
a device driver for contacting and longitudinally moving the elongated delivery catheter loaded within the working channel;
characterized in that the loading port is configured to receive a cassette having the delivery catheter coiled therein and a length of the delivery catheter extending out of the cassette and disposed within the working channel.

Optionally, the coiled delivery catheter is spring biased to unwind and has a releasable restraining element to prevent the coiled delivery catheter from unwinding.

Optionally, a driving element is provided that engages the length of the delivery catheter extending out of the cassette and acts to urge the catheter down the working channel.

Optionally, the driving element has a crank to urge the delivery catheter down the working channel.

Optionally, the driving element includes a friction wheel.

Optionally, the driving element is finger operable.

These and other advantages of the various embodiments of the invention will become more apparent from the following detailed description and the accompanying exemplary drawings.

### BRIEF DESCRIPTION OF THE DRAWINGS

Figure 1 is an elevational view of an endoscope assembly present only to assist in the understanding of the invention including an endoscope and an outer sheath.
Figure 2 is a transverse cross-sectional view taken along the lines 2-2 of the endoscope shown in Figure 1
Figure 3 is a transverse cross-sectional view taken along the lines of 3-3 of the endoscope assembly shown in Figure 1 with the endoscope portions (which would be the same as Figure 2) removed for clarity.
Figure 4 is a front view of the reproductive organs of a female patient with the inflatable member of the sheath of the endoscope in an inflated configuration in within the patient's uterine cervix.
Figure 5 is a perspective view, partially in section, of an endoscope having a loading port and friction thumbwheel for driving an elongated medical device within the working channel of the endoscope.
Figure 6 is a transverse cross-section of the endoscope of Figure 7 taken along lines 6-6.
Figure 7 is a perspective view of an endoscope with pistol-grip configuration for one-handed operation with a driving member in the handle portion of the endoscope for advancing an elongated medical device within the working channel of the endoscope.
Figure 8 is a cross-section of the endoscope shown in Figure 7 taken along the lines 8-8.
Figure 9A is a perspective view of a cassette with a delivery catheter;
Figure 9B is a perspective view of the delivery catheter of Fig 9A uncoiled from the cassette;
Fig. 9C is a perspective view of the cassette with delivery catheter shown in Figure 9A in conjunction with a flexible hysteroscope;
Figure 10 is an elevational view of a trigger mechanism deployment tool with an endoscope shown in phantom.
Figure 11A is an elevational view of a trigger mechanism present only to assist in the understanding of the invention with a first attachment configuration.
Figure 11B is an elevational view of a trigger mechanism present only to assist in the understanding of the invention with a second attachment configuration.
Fig. 12A is an elevational view of a ribbon restraining a self- expanding occluding contraceptive device;
Fig. 12B is an elevational view of the restraining device of Fig. 12A with the restraining ribbon partially unwound from around the self expanding occluding contraceptive device;
Fig. 12C is an elevational view of the restraining device of Fig 12B with the restraining ribbon fully unwound and the self expanding occluding contraceptive device in the expanded state;

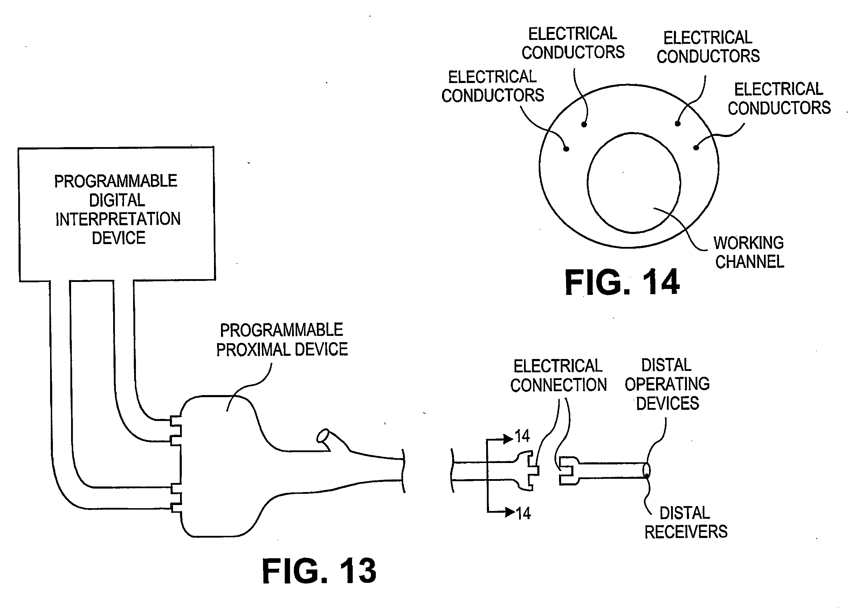

Fig. 13 is an elevational view of a digital scope with attachable distal section;
Fig 14 is a transverse cross section of Fig. 13 taken along the line 14-14;
Fig. 15 is a perspective view of a dual-action, dual-trigger, clip- on activation mechanism present only to assist in the understanding of the invention;
Fig. 16 is an expansion of the drawing contained within the dashed circle shown on Fig. 15;
Fig. 17 is an elevational view of the embodiment of Fig. 15 installed on a catheter of Fig. 12;
Fig. 18 is an elevational view of a scissor-handled device present only to assist in the understanding of the invention;
Fig. 19 is an elevational view of another scissor-handled device present only to assist in the understanding of the invention.
Fig. 20 is an expanded view of the portion of Fig 19 contained within the circle indicated in Fig. 19.

### DETAILED DESCRIPTION OF EMBODIMENTS OF THE INVENTION

The present invention provides devices and systems for delivering medical devices into a patient, particularly occlusive contraceptive or sterilization devices within a female patient's fallopian tube.

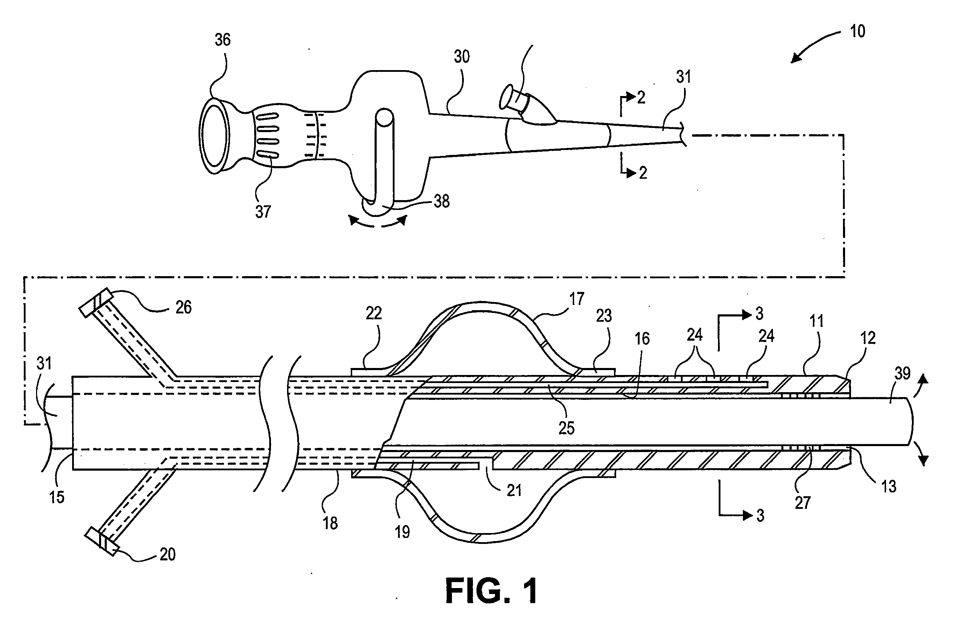

Figures 1-3 show an endoscope assembly 10 which includes an elongated sheath 11 which surrounds a length of an endoscope 30 and which is suitable for viewing or treating a female patient's uterus or fallopian tube. end, a proximal end 14, and a port 15 in the proximal end. A first inner lumen 16 extends within the elongated shaft 11 between the ports 13 and 15 and is configured to receive the endoscope 30 which is to be used. An expandable member 17, e. g. an inflatable balloon, is disposed about the elongated sheath 11 on the distal portion 18 of the sheath 11. A second inner lumen 19 extends within the shaft 11 from inflation port 20 to the discharge port 21 which opens to the interior of the balloon 17. The proximal and distal ends 22 and 23 of balloon 17 are secured to the shaft 11 by a suitable adhesive, fusion bonding or other conventional techniques.

The sheath 11 is preferably provided with ports 24 distal to the balloon 17 which is in fluid communication with third inner lumen 25 which extends to the port 26 at the proximal end of the shaft 11. The one or more ports 24 may be employed to withdraw fluid from or inject fluid into the patient's body cavity or lumen in which the sheath 11 is deployed. Alternatively, the at least one port 24 may be in fluid communication with the first lumen 16. Ports 24 facilitate drainage out of the patient's uterus and fluid flow into the uterus. The distal end of the elongate sheath 11 may have a seal 27 around the shaft of the endoscope 30 to prevent fluid from entry into the first inner lumen 16 of the sheath 11.

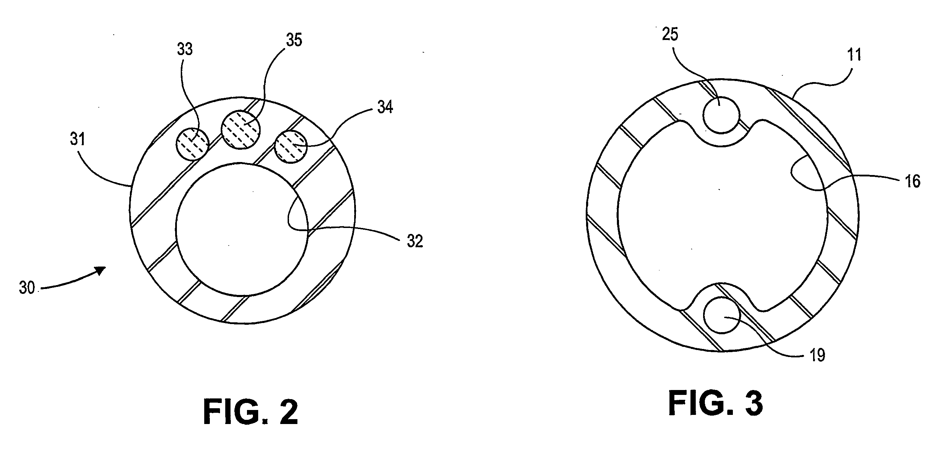

Figures 1 and 2 illustrate details of the endoscope 30 which has an elongated shaft 31, a working channel 32 extending through the elongated shaft, and optical fibers 33 and 34 for transmitting light to the distal end of the endoscope and optical fiber 35 for transmitting images from the distal end to an eye-piece 36 (or a camera or other device) at the proximal end of the endoscope. A focusing ring 37 is provided distal to the eye-piece to focus a lens (not shown) at the distal end of the sheath. Deflecting lever 38 allows the operator to move the lever (arrows at lever) to deflect the distal end 39 of the endoscope (arrows at tip).

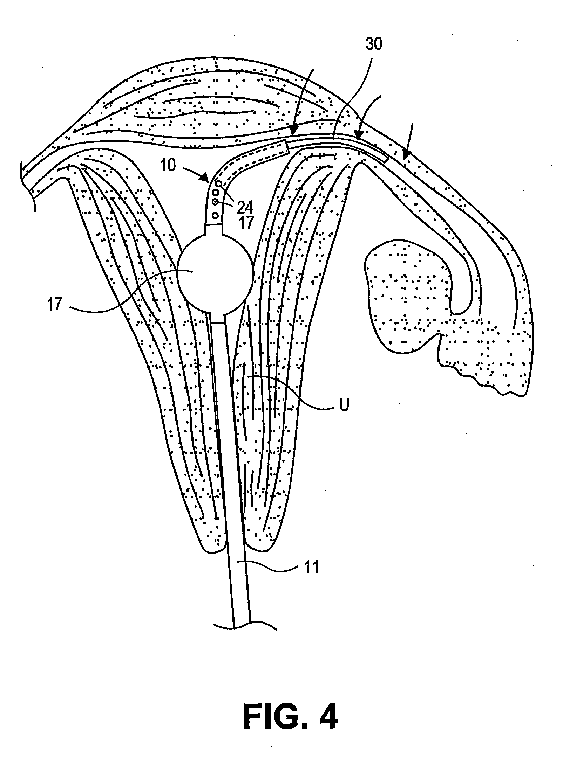

The sheath 11 has a first inner lumen 16 which encases the elongate shaft 31 of the endoscope 30 when the endoscope 30 is delivered to the patient's body cavity or lumen, e.g. uterus. The inflatable member 17 may be in a deflated configuration when the endoscope assembly 10 is inserted through the patient's cervix to the interior of the patient's uterus. When the assembly is deployed within the patient's uterus, inflation fluid is introduced through the proximal end of the second inner lumen 19 through port 20, the to expand inflatable member 17 to contact and seal against the patient's uterine cervix as shown in Figure 4.

The inflatable member 17 is formed of relatively non-compliant biocompatible polymeric material such as polyethylene terephthalate, nylon and the like to facilitate inflation to a predetermined size or it is formed of a more compliant material such as polyethylene Hytrel and the like to adjust the size of the balloon by the amount of inflation pressure used.

One of the advantages of a flexible, small diameter hysteroscope is the ability of the scope to be used in examining most uteruses without the need to pull the cervix with a tenaculum to straighten the uterus. That is, typically when a stiff, generally larger diameter scope is used, the cervix must be grasped and pulled, for example by a tenaculum, to straighten the uterus sufficiently to insert the scope and view the uterus interior without being blocked by the uterus wall of a curved uterus. This is a very uncomfortable procedure and in order to do it, the gynecologist generally must hospitalize and anesthetize the patient. In these situations, the relative rigid, large diameter scope shaft itself might provide sufficient blockage of the cervix to facilitate insufflation or irrigation. If so, however, drainage of the uterus might not be possible with the large diameter shaft in place. A suitable flexible hysteroscope is the hysteroscope sold by Olympus America, Inc. (model HYF-XP).

The endoscope assembly 10 provides the ability to perform insufflation and drainage using a small diameter and flexible hysteroscope, which in turn allows the procedure to take place in the doctor's office or a medical suite without the need for hospitalization. The endoscope assembly 10 may come in a variety of sizes in length, inflatable member size, and diameter that may be selected as appropriate for the individual patient or for the particular procedure.

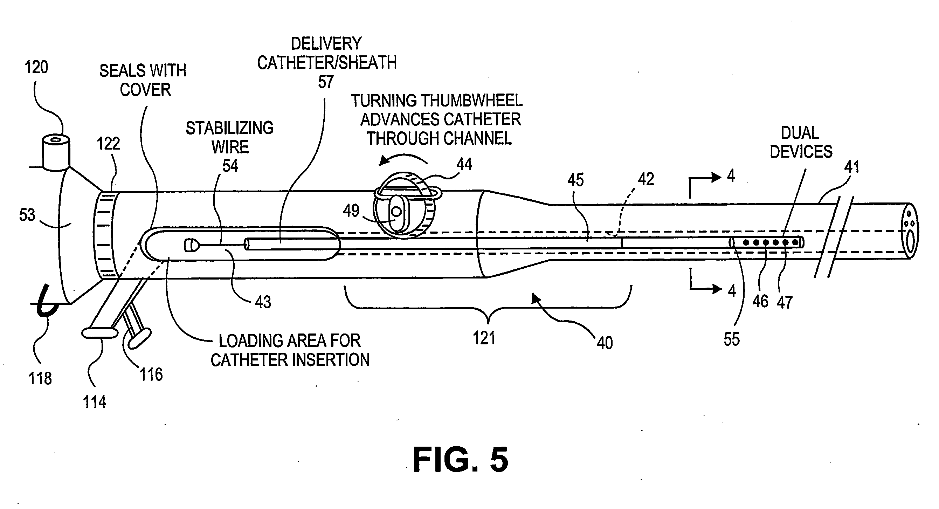

Figure 5 illustrates an endoscope 40 embodying features of the invention. The endoscope 40 has an elongate shaft 41, a working channel 42 and a loading port 43 providing access to the working channel 42. A driving device 44 such as the thumbwheel shown facilitates longitudinal movement of an elongated delivery catheter 45 within the working channel 42. The device driver 44 shown is manually operated but it may be motor driven.

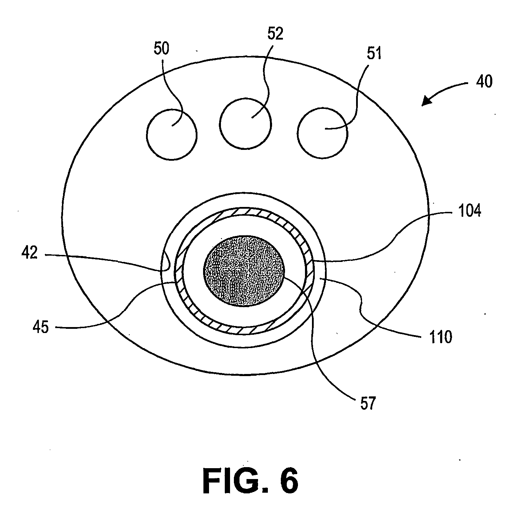

Figures 5 and 6 illustrate a delivery catheter 45 for delivery of occluding contraceptive devices 46 and 47. The driver 44 is built into the scope for desired manipulation of the delivery catheter 45 loaded through the loading port 43. In Figure 5 the fingerwheel 44 is intended to advance the delivery catheter down the working channel 42. As shown in Fig 5 the thumbwheel 44 may be slidably disposed within slot 49 so that the outer periphery of the thumbwheel 44 can be raised to allow the delivery catheter 45 to be inserted into the working channel 42. The thumbwheel 44 can then be pressed against the exterior of the delivery catheter 45 and rotated to advance or retract the delivery catheter within the working channel 42.

A cover may be provided on the loading port 43 that may be closed and sealed tightly so that the working channel 42 may be securely closed to form a fluid tight channel if desired. The working channel 42 may also be in fluid communication with a proximal access port. Thus if the working channel 42 is not being used for an instrument or medical device such as a delivery catheter 45, it may be supplied with an irrigation fluid or fluid may be drained from the uterus. The endoscope 40 is provided with fiber optics 50 and 51 for transmission of light to the distal tip of the scope and fiber optic 52 for image transmission to the proximal end. Eye-piece 53 is provided on the proximal end to facilitate viewing the site distal to the distal end of the scope. The delivery catheter 45 has a stabilizer wire 54 with a plunger 55 on the distal end to hold the occlusion elements 46 and 47 while the delivery catheter sheath 57 is withdrawn so as to deploy the occlusion elements.

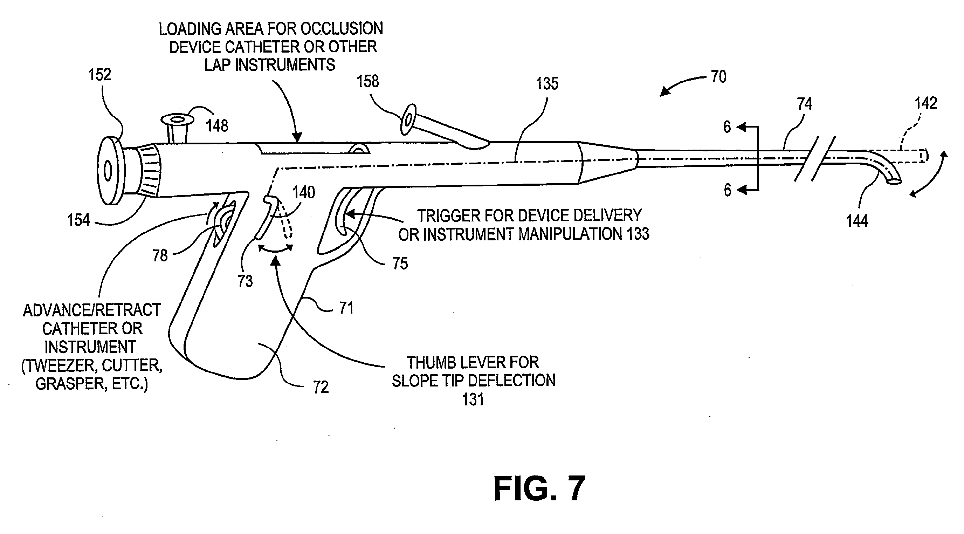

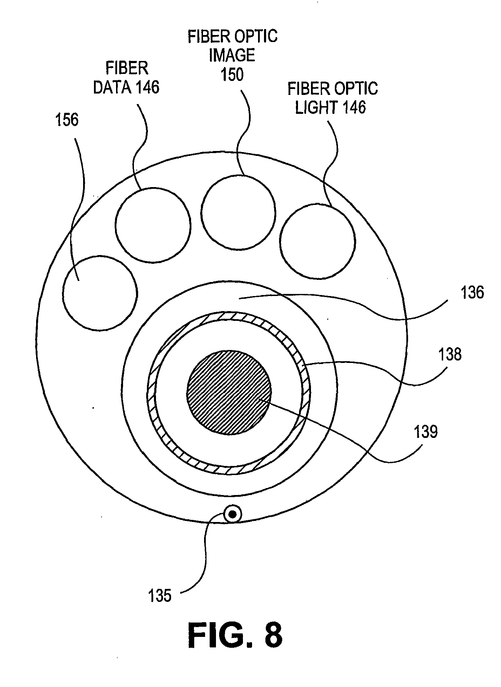

Figures 7 and 8 illustrate an alternative design for an endoscope 70 which is provided with a pistol grip handle 71 having a palm engaging portion 72, a lever 73 to deflect the distal portion of the elongated shaft 74 as shown and a trigger 75 for delivery or manipulation of a delivery catheter (not shown) which would be disposed within working channel 77 and a thumbwheel driver 78 which is configured to engage the delivery catheter and longitudinally move the catheter within the working channel 77. The lever 73, the trigger 75 and the thumbwheel driver 78 are shown located on the pistol grip handle 71. In this example the mechanism of trigger 75 activates the delivery catheter to deposit an occluding device by withdrawing the catheter sheath from over the occluding device. The occluding devices may be placed in the fallopian tubes for enhancing tissue growth into the occluding devices for purposes of contraception by delivery catheters described in the application Serial No. 10/746,131, filed on December 24, 2003, entitled "Contraceptive Devices and Delivery Systems".

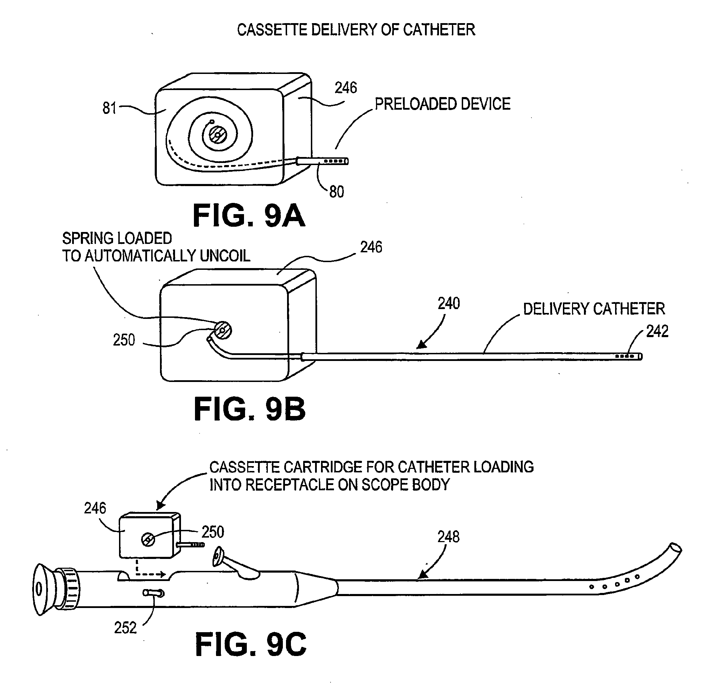

As shown in Figs. 9A, 9B, and 9C an elongated medical device 80 such as a delivery catheter for fallopian tube occlusion elements may come packaged in a cassette 81 for convenient storage, handling and mounting on an endoscope. A delivery catheter 80 with one or more occluding devices 83 pre-loaded within an inner lumen of the catheter can be provided in a cartridge form with the catheter coiled around a central post within the cassette 81.

The delivery catheter 80 may be coiled about spring loaded shaft within the cassette 81 urging the delivery catheter to be expelled from the cassette, but releasably retained, for example by a restraining element (not shown) which is broken by the act of installing the cassette into the scope. When the cassette 81 is installed in the endoscope, the restraint is removed (e. g. A paper tape is broken or a sticky tape is removed) the catheter is released so that it is free to unwind. As it uncoils, the catheter is projected down the working channel of the scope.

Alternatively, the delivery catheter 80 may be wound onto a central hub (not shown) which has a crank which moves the delivery catheter and the operator may project the catheter assembly down the working channel by turning the crank.

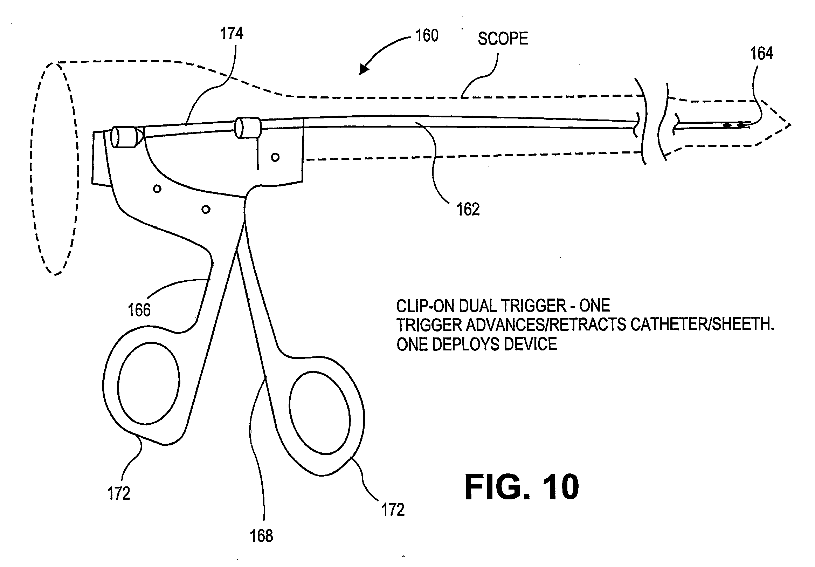

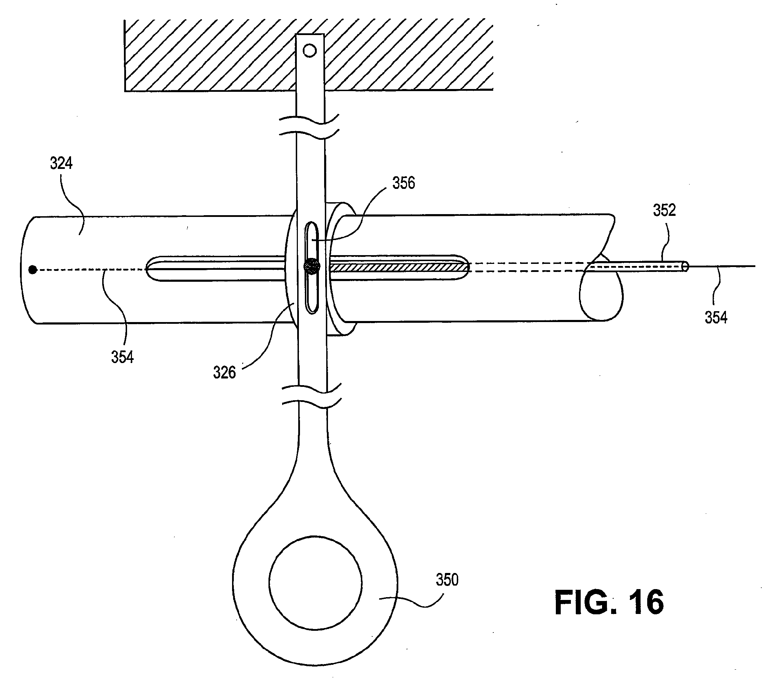

As seen in Figs. 10, 11A, 11B and 15-20, the delivery catheter may be loaded into a specialized tool including trigger mechanism for use with an endoscope such as a hysteroscope. This trigger mechanism may be in the form of two triggers, each with different and specialized function, e. g. where one trigger advances and retracts the entire catheter assembly and the other trigger activates the extrusion of the occluding device from the catheter lumen, or the dual action may be provided in the same trigger where one attachment configuration moves the entire catheter assembly forward and backward for positioning the catheter, and the other attachment configuration acts to extrude the occluding device. The one trigger/two action example may be switched from the one attachment configuration to the other in any number of means including a switch, moving pins, detachment and reattachment in a different configuration or the like.

The trigger mechanism may be built into the scope, or may be designed to clip onto the scope if the scope is not provided with a built in multi-trigger mechanism.

Referring to Fig. 10 a scope 160 is loaded with a delivery catheter 162 with occluding device or devices 164 in the distal end of the catheter. The trigger arrangement may be a handle (not shown) with a trigger 166 or may be a set of finger levers 166, 168. It is generally preferable to have a finger hole 172 on the end of the trigger lever rather than having a flat trigger. The finger hole is useful to facilitate moving the trigger lever both backward and forward rather than a trigger motion primarily in only one direction. This greater flexibility is advantageous and is greatly preferred by the physicians; it allows the application of more precise movement and generally greater skill by the operator and is similar to other medial tools for use in similar procedures.

In the two-trigger configuration, one trigger 168 is attached so that moving the trigger forward and back moves the entire catheter and stabilizing wire assembly forward and back. Moving the other trigger 166 moves only the catheter outer wall 162 backwards while the stabilizing wire is held stationary. As previously described, this relative motion between the catheter outer wall and the stabilizing wire acts to lay down the occluding device into the desired location by withdrawing the sheath from over the occluding device.

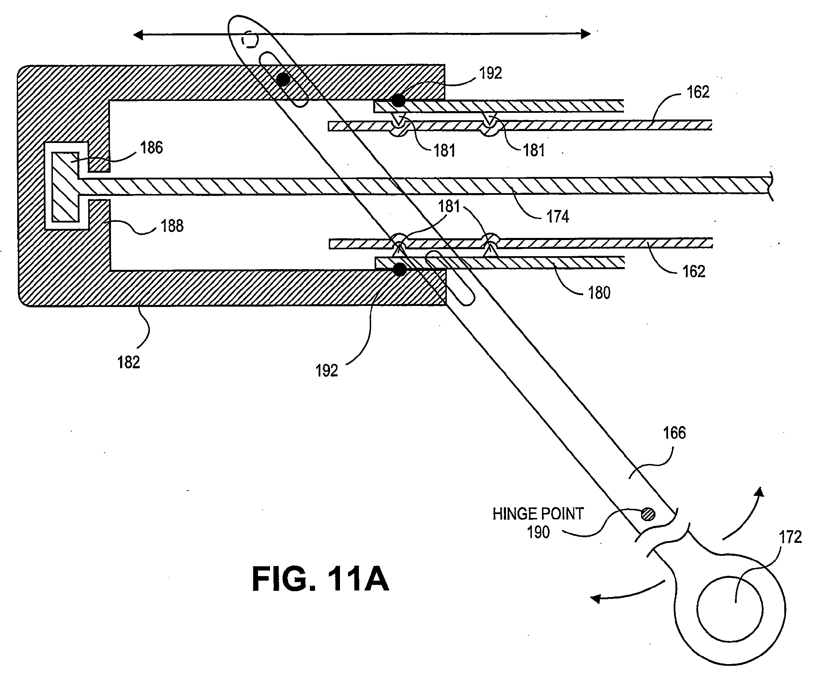

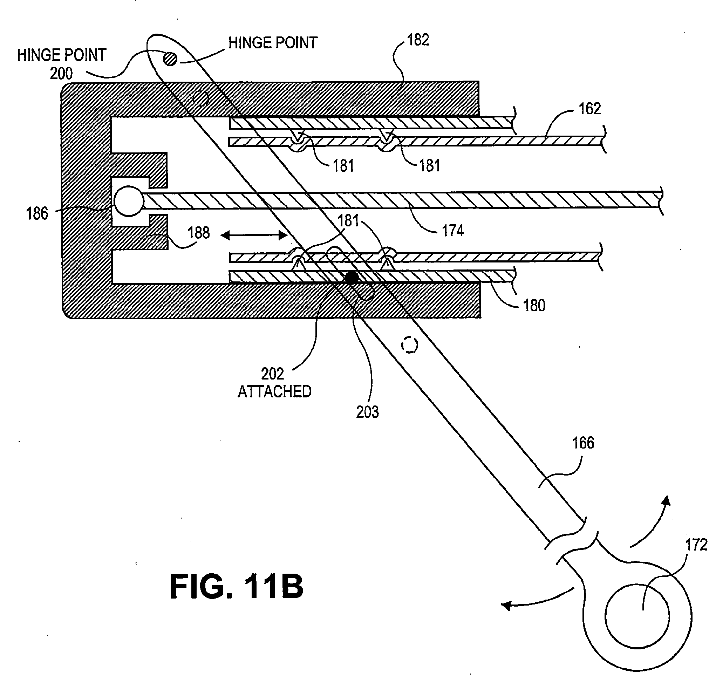

In the device with only one trigger, the trigger has two configurations and is transformable between the two configurations to facilitate different actions by the same trigger motion. Reference is made to Figs 11A and 11B. The catheter 162 is placed into the working channel of a scope. The proximal portion of the catheter is attached into an inner receiving unit that may be in the shape of an inner cylindrical sleeve 180. This may be by any conventional fastening means, for example snapping the deformable plastic into rigid barbs or detents 181. The stabilizing wire 174 is attached to an outer receivable assembly which may be in the form of an outer cylindrical sleeve 182, for example by snapping a terminal button 186 on the proximal end of the stabilizing wire into a retaining block 188. The inner receiving unit is slidable within the outer receivable unit.

Initially, as shown in Fig 11A, the trigger lever 166 is hinged at hinge point 190 and attached to the outer assembly for example by attachment pin 194. The inner and outer sleeves are releasably attached, for example by ball detents 192. Movement of the trigger forward and backward as shown by the arrows near the finger grip 172 causes the entire catheter/stabilizing rod assembly to move forward and backward. If the trigger lever 166 is pulled backward, the catheter is pushed out forward from the scope. If the trigger is moved forward, the catheter is withdrawn. In this way the trigger may be used to position the catheter assembly by pushing it further out the end of the scope or withdrawing back into the scope. The distal end of the trigger lever is attached to the outer cylinder 182 by pin 194 which rides in a slot 195 so that the catheter assembly may move forward and backward in a straight line as the trigger lever is pulled back or forward and trigger lever is rotated around the rotation point.

In the second configuration, as shown in Fig. 11B, the attachments and hinge points have been changed, so that the trigger lever is now hinged at new hinge point 200 and attached, not to the outer cylinder 182, but now to inner cylinder 180. The attachment may be, for example at pin 202 in slot 203. The outer cylinder is fixed, for example by fixed attachment to the scope handle. When the trigger lever is pulled, the releasable attachment between the inner cylinder and the outer cylinder is broken free, for example friction attachment created by the ball detents is overcome, and the inner cylinder slides within the outer cylinder, pulling the catheter outer sheath back relative to the stabilizing rod.

In practice, the catheter is placed into the working channel of the scope with the proximal portion of the catheter snapped into barbed retaining surface features in an inner cylinder, and the stabilization wire with a terminal button 186 fed through the outer cylinder and the terminal button snapped into retaining block 188. The trigger is hinged at hinge pin 190, and attached to the outer cylinder by attachment pin 194 in slot 195. When the scope is inserted into the uterus of a patient and the tip approaches the fallopian tube, the trigger mechanism may be used to advance and retract the catheter assembly as a whole to place the catheter shaft properly into the fallopian tube. When properly placed, the configuration of the trigger mechanism is transformed to the second configuration, for example, the hinge point at 190 is unattached, the trigger lever is reattached at pin 202 in slot 203, the pin 194 is unattached, and the trigger lever reattached at hinge point 200. Pulling the trigger lever then breaks the releasable attachment between the inner and outer cylinder and pulls the inner cylinder with the catheter sheath backward relative to the stabilizing wire. This then has the effect of sliding the catheter sheath back from over the occluding device and depositing the occluding device into the fallopian tube at the desired location.

The transfer of the attachment points and hinge points may be done in one motion by, for example, a toggle switch that pulls the attachment pin 194 and hinge pin 190 and inserting hinge pin 200 and attachment pin 202. Thus the trigger mechanism may be in the first configuration initially, and once the catheter is located with the occluding devices at the desired location, the switch can be thrown placing the trigger mechanism in the second configuration. The trigger can then be used to deposit the occluding devices.

Besides a toggle switch, another scheme for switching from catheter advancement to device deployment configuration would be to have the two different sides of the trigger fitted with pins that fit the holes in the first configuration (e.g. pins 190, 194) pointing out one direction and the pins that fit the holes for the other configuration (e.g. pins 200, 203) pointing out the other direction. The trigger could change function merely by turning it over and thus removing the pins pointing out in that direction from their mating holes and then inserting the pins pointing in the other direction into their mating holes. This functionality is not specifically illustrated in the attached drawings although it may be achieved using the elements illustrated.

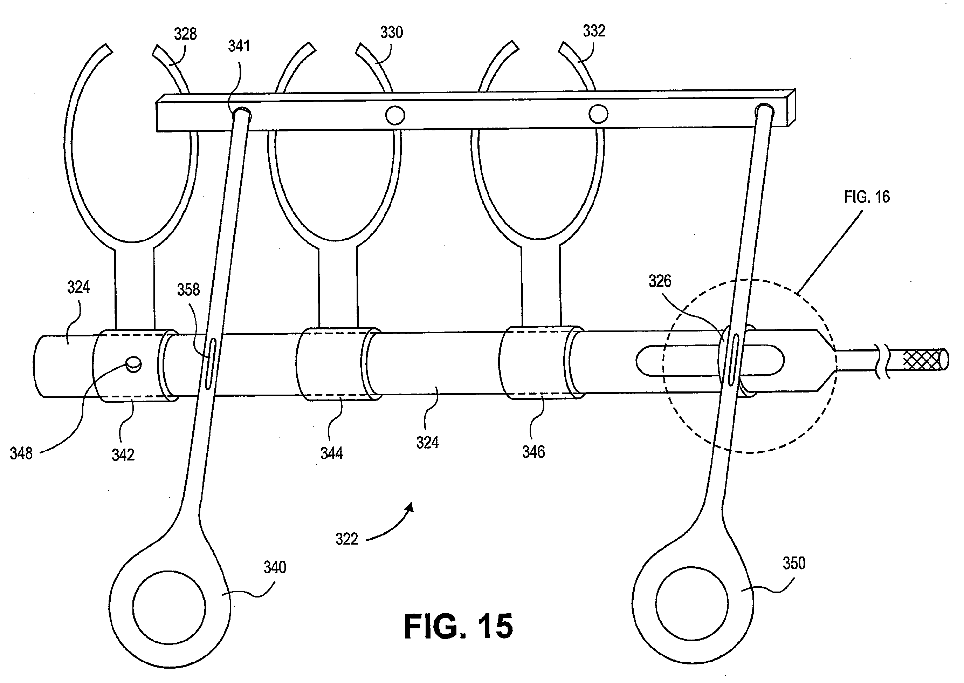

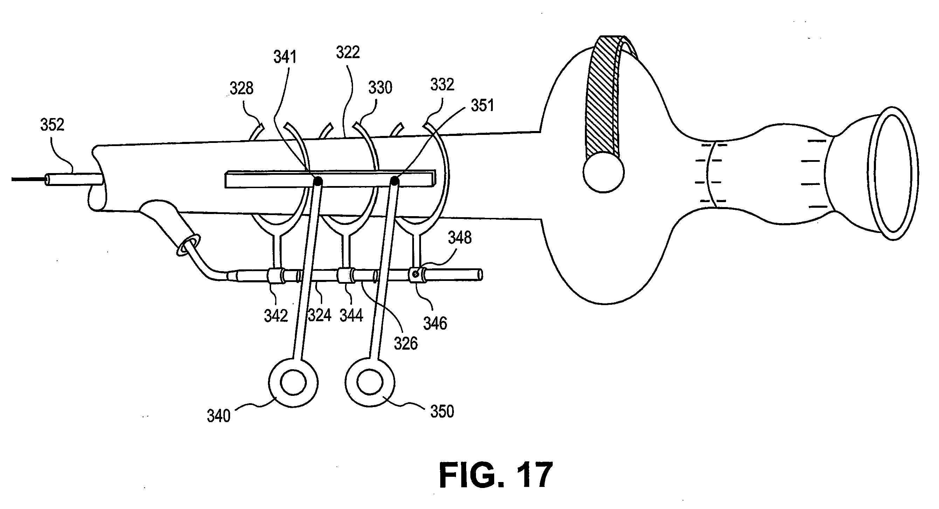

Similarly, the dual trigger function may be provided by two separate trigger levers, which may be provided in a clip-on catheter delivery system as shown in Figs 15-17. A dual trigger clip-on bracket 322 may be provided as show in Fig. 15 to hold the handle 324 at the proximal end of the delivery catheter, including the slider ring 326 for delivery of the occluding devices. The clip-on holder with dual triggers is shown in place on the scope handle as shown in Fig 17, with the delivery catheter shaft extending through the side port 320 into the working channel and thus to the distal end of the scope 310. Three clips, 328,330 and 332 are spring biased in the closed direction so that they may be clipped onto the handle portion of the scope and be frictionally fixed at that location. The trigger 340 attached to the handle of the delivery catheter 324 may then be moved forward and backward in a longitudinal direction slidably moving the entire catheter assembly forward and backward by sliding the handle within the cylinders 342, 344 and 346. (Note that the trigger levers are in reversed position in Fig 15 and Fig 17. This is merely different and equivalent construction of the invention). By deflecting the end of the scope and thus pointing it toward the desired location, for example pointing it at the ostium of a fallopian tube, and then moving the appropriate trigger, the catheter may be advanced into the desired position.

Once the catheter is in position, it may be fastened to the scope to prevent further sliding within the cylinders, by example using a setscrew, 348. The second trigger 350 may then be pulled back to withdraw the catheter 352 relative to the stabilizing wire 354 to deploy an occluding device or devices as previously described.

The slider ring 326 is attached to the trigger 356 by means of a pin on the slider ring passing through a slot 356 so that rotating motion of the trigger may be translated into longitudinal motion of the slider ring. A similar attachment between trigger 340 and the handle using slot 358 allows the straight longitudinal motion by rotation of trigger 340.

In use, as shown in Fig 17, the catheter handle 324 is placed into the slideable cylinders 342, 344, 346, and the catheter shaft is loaded into the side port and thus into the working channel of the scope. When the tip of the scope is at the desired location and pointed in the desired direction, the catheter shaft may be advanced and retracted longitudinally by rotating trigger 340 around rotation point 341. When the catheter shaft is has thus been advanced to the desired location, for example placed at the correct depth into a fallopian tube of a patient, then the handle of the catheter may be firmly secured in the slidable cylinders (for example by a set screw, pin, or clamp or the like, not shown) so that it is no longer able to slide within those cylinders. It is desirable to have a simple means of firmly fixing the handle to prevent longitudinal motion while having a convenient means of seeing that the handle is in the secured condition. Therefore a color-coded clamp that has a visible signal that indicates that it is firmly clamping the handle (not depicted) is suggested.

Once the handle has been secured against any further longitudinal motion, the other trigger 350 may be employed to cause relative longitudinal motion between the catheter shaft, acting as a sheath over the occluding device at the distal end, and the stabilizing wire 354 which holds the occluding device in place while the sheath is withdrawn. The trigger 350 is pulled, rotating it around rotation point 351 and thus pulling it proximally to slide the sheath from over the occluding device, thus depositing it in the fallopian tube of the patient

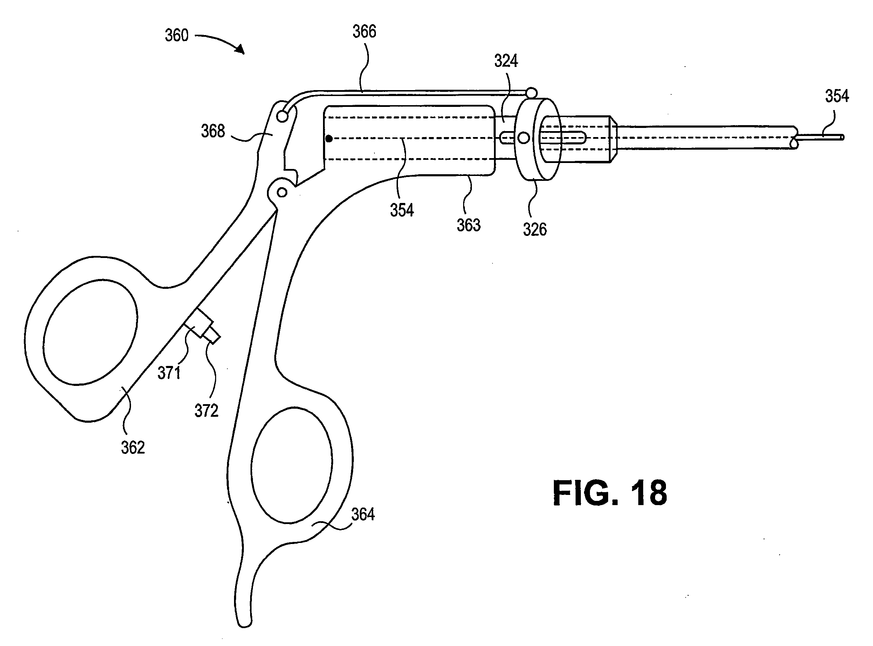

A number of equivalent embodiments may be made. For example, in Fig. 18, an instrument 360 with scissor type finger levers 362, 364 of the type typically used in instruments for performing MIS (Minimally Invasive Surgery) with various types of endoscopes can be adapted for use with a delivery catheter. The delivery catheter handle 324 is loaded into the instrument body 363, with the stabilizing wire 354 attached to the proximal end of the delivery catheter handle. A sliding ring 326 is attached to the catheter tube but the stabilizing wire runs through the catheter tube and out, and all the way to the proximal end of the handle where it is fastened.

A pull wire 366 or the like is attached between the base arm 368 of the instrument and the sliding ring 326. The delivery catheter is inserted through the side port of the scope into the working channel of the scope, and moving the instrument forward and back will push the entire catheter assembly forward and backward within the working channel. The instrument is attached to a channel in the scope handle and rides forward and backward in a longitudinal direction (not shown) or be otherwise guided in a longitudinal direction, or may be freestanding. When the delivery catheter tip has been located as desired, the operator may firmly attach the instrument to the scope, for example by clipping or otherwise attaching the instrument body 363 onto the scope handle. The operator may then squeeze the scissor handles 362,364 together thus pulling the pull wire 366 which pulls the slider ring 326 proximally relative to the stabilizing wire 354. This pulls the distal catheter sheath from over the occluding device and it is thus laid down in the desired location as previously detailed. Since the occluding device may be a self expanding stent-like device, it is desirable not to try to push it longitudinally in the fallopian tube once it has be uncovered from the catheter lumen since it will then expand to contact the interior surface of the fallopian tubes, and the fallopian tubes tend to be rather delicate and could be injured by pushing the occluding device longitudinally along the fallopian tube.

If more than one occluding device is loaded into the delivery catheter, the scissor like handles on the instrument may be provided with two stops 371,372 such that the handle can be activated twice, once only as far as permitted by stop 372, then the stop can be removed, and the next time the instrument is operated by squeezing the finger levers as far as permitted by stop 371, a second relative movement of the catheter tube and the stabilizing wire will lay down the second occluding device. In this way the amount of movement for the deployment of each occluding device may be pre-set. Although illustrated with two stops, as many stops may be employed as required for the number of occluding devices pre-loaded into the delivery catheter.

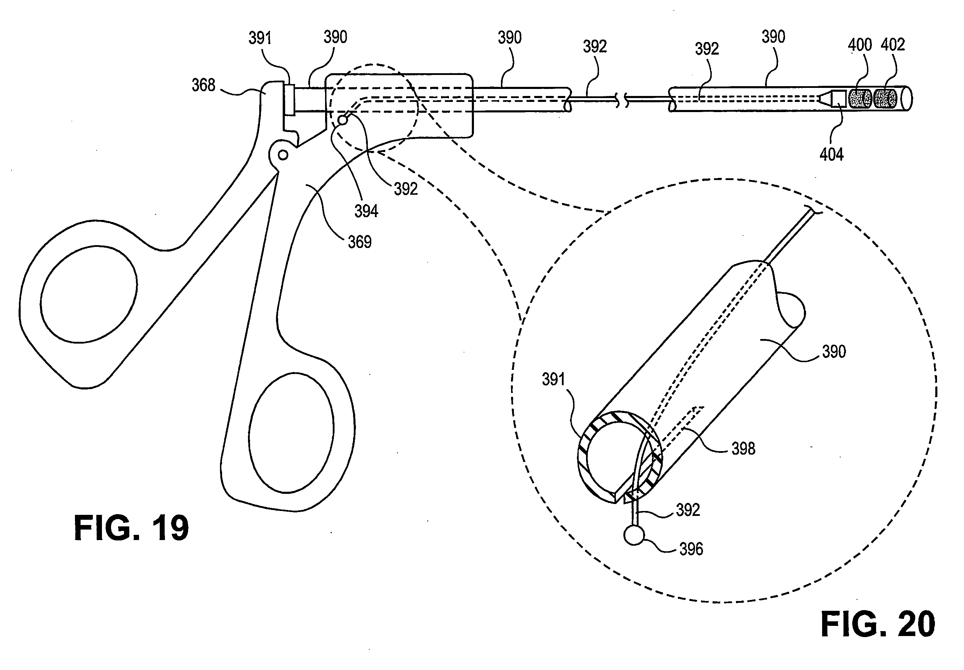

A similar example is depicted in Figs. 19-20 wherein the delivery catheter does not have a handle and the catheter with the stabilizing wire attaches directly to the instrument. In this example, the catheter tube 390 attaches directly to the base arm 368. The terminal end of the sheath may be a ring 391 or the like that can easily fit into a holder depression to firmly attach the sheath of the delivery catheter to the base arm, or the retaining structure in the base arm may have barbs or the like as described for holding the catheter tube in the previous example. The stabilizer wire 392 has a proximal plug 394 that attached to the second base arm 369. The stabilizing wire goes through a peel-away slit 398 in the catheter tube which is near and just distal to the terminal plug. When the finger levers of the instrument are squeezed together, they pull the catheter tube proximally relative to the stabilizing wire, and the stabilizing wire is pulled through the peel-away slit, allowing the catheter tube to be withdrawn and at the distal end of the delivery catheter assembly slid over the occluding devices 400, 402 which are restrained by terminal restraining plug 404 attached to the stabilizing wire. Thus the occluding devices are laid down out of the distal end of the catheter tube.

Scopes as currently configured generally allow visualization of the surface of the tissue near the end of the scope. They transmit light out the distal end of the scope, for example by light transmitting fibers illuminated at the proximal end of the scope, and provide a lens and light transmitting fiber to carry the illuminated image at the distal end of the catheter to an eyepiece or camera at the proximal end of the scope. However this allows the operator to visualize only the surface of the tissue at the distal end of the scope. It would be advantageous to be able to obtain information about the tissue features below the surface.

One method that would provide this, especially for hysteroscopes, would be an ultrasound transducer designed for use through the working channel of the hysteroscope. However, one of the problems with the ultrasonic transducers is that the expensive portion of their mechanism may not be disposable. Moreover, generally the ultrasonic transducers should not encounter tissue in use, because the complex electronics involved is difficult to sterilize. The method generally used to overcome this problem is to place the device in a sterile, disposable sheath. Such a sheath could be provided that covers the transducer within the working channel, and still allows movement within the working channel of the scope. Alternatively, the delicate parts could be built in to the scope so that direct contact with the tissue is not necessary, i.e. the scope itself acts as the sterile sheath over the device Alternatively, if the scope is a clip-on digital scope as described immediately below, the portion with the ultrasonic transducer can be programmed to function based on the multi-purpose electrical conductors, and be removed for convenient sterilization.

If the scope is a digital device, the distal end portion may be a clip-on portion that may provides a very specific function. The proximal portion has conductors running therethrough and terminates in ends in electrical connections. These conductors carry electrical signals only, and thus can send different signals to the end of the scope depending on the desired function and the programming being used to generate those signals, and can receive and process any number of different signals, again depending on the programming of the receiving device.

For example, one type of distal portion may contain light emitting diodes and an image-sensing portion. For example, the scope may be programmed to send an electrical signal down electrical conductors one and two to activate the LEDs. A light sensing device may then generate a signal and transmit that signal back to a microprocessor attached to the scope, and the microprocessor interprets the image from that signal. Thus a "visual" image may be received from that type of clip-on distal end.

The same three electrical conductors may clip on to a different end, for example the ultrasonic transducer mentioned above. The proximal portion is programmed to send a signal to activate the ultrasonic transmitter, and perhaps another signal to move the transducer if appropriate, and a third electrical conductor is attached to the receiver to receive the return ultrasonic signal. That signal is transmitted to the microprocessor to form an image that can be viewed and interpreted.

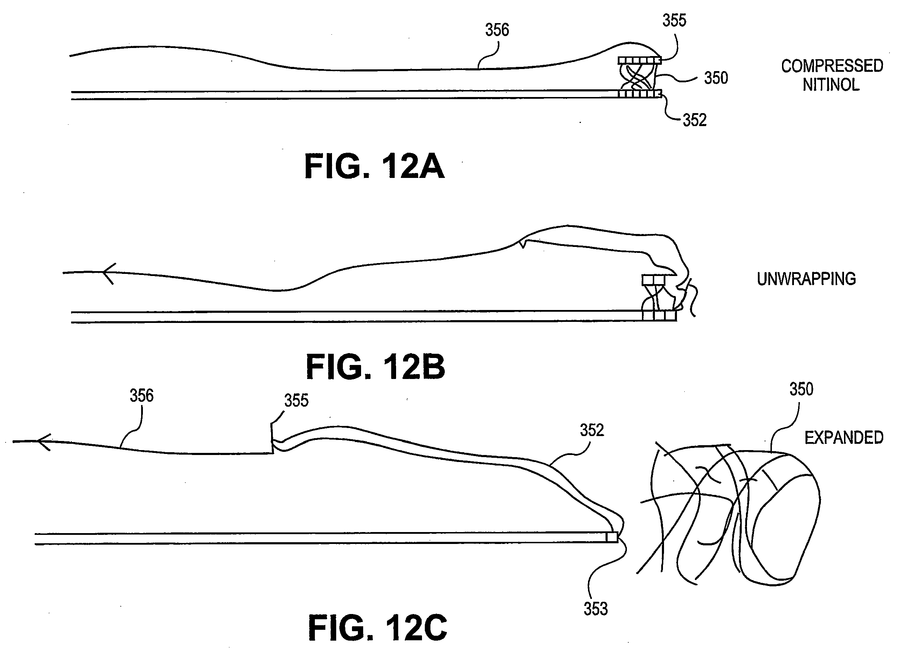

As illustrated in Fig. 12A-12C, an alternative method of releasing a restrained selfexpanding stent-like occluding device, for example a nitinol stent with fibers therein 350 may be provided in the form of a wrapping ribbon. The occluding device would, be tightly wrapped as seen in Fig 12A by a ribbon 352 at the distal end of a push rod 354. The ribbon would be attached at its proximal end 353 to the push rod, and at the distal end 355 to a pull string 356. When the stent has been positioned at the desired location, for example within a patient's fallopian tube, the pull string may be pulled proximally, unwinding the constraining ribbon and thus releasing the occluding device. The self-expanding occluding device would then be free to expand to its desired diameter.

## Claims

1. An endoscope (40, 70, 82) comprising:
an elongate shaft (41, 74);
a working channel (42, 77) extending through at least a portion of the elongated shaft;
a loading port (43) in a proximal portion of the elongated shaft for loading an elongated delivery catheter (80) within the working channel; and
a device driver (44, 78) for contacting and longitudinally moving the elongated delivery catheter (80) loaded within the working channel;
**characterized in that** the loading port is configured to receive a cassette (81) having the delivery catheter (80) coiled therein and a length of the delivery catheter extending out of the cassette (81) and disposable within the working channel.

2. The endoscope of claim 1, wherein the coiled delivery catheter (80) is spring biased to unwind and has a releasable restraining element to prevent the coiled delivery catheter from unwinding.

3. The endoscope of claim 2, wherein a driving element is provided that engages the length of the delivery catheter extending out of the cassette and acts to urge the catheter down the working channel.

4. The endoscope of claim 3, wherein the driving element has a crank to urge the delivery catheter down the working channel.

5. The endoscope of claim 3, wherein the driving element includes a friction wheel.

6. The endoscope of claim 5, wherein the driving element is finger operable.

## Patentansprüche

1. Endoskop (40, 70, 82), welches umfasst:
einen langgestreckten Schaft (41, 74);
einen Arbeitskanal (42, 77), der sich durch mindestens einen Abschnitt des langgestreckten Schafts erstreckt;
eine Beschickungsöffnung (43) in einem proximalen Abschnitt des langgestreckten Schafts, um das Innere des Arbeitskanals mit einem langgestreckten Zuführungskatheter (80) zu beschicken; und
einen Gerätetreiber (44, 78) zum Kontaktieren und Bewegen des beschickten langgestreckten Zuführungskatheters (80) in Längsrichtung im Innern des Arbeitskanals;
**dadurch gekennzeichnet, dass** die Beschickungsöffnung dazu konfiguriert ist, eine Kassette (81) aufzunehmen, in welcher der Zuführungskatheter (80) aufgewickelt ist und eine Länge des Zuführungskatheters aus der Kassette (81) herausragt und im Innern des Arbeitskanals verfügbar ist.

2. Endoskop nach Anspruch 1, wobei der aufgewickelte Zuführungskatheter (80) zum Abwickeln federvorgespannt ist und ein lösbares Spannelement aufweist, um ein Abwickeln des aufgewickelten Zuführungskatheters zu verhindern.

3. Endoskop nach Anspruch 2, wobei ein Antriebselement bereitgestellt ist, das die aus der Kassette herausragende Länge des Zuführungskatheters erfasst und dazu dient, den Katheter den Arbeitskanal hinunter zu treiben.

4. Endoskop nach Anspruch 3, wobei das Antriebselement einen Hebel aufweist, um den Zuführungskatheter den Arbeitskanal hinunter zu treiben.

5. Endoskop nach Anspruch 3, wobei das Antriebselement ein Reibrad beinhaltet.

6. Endoskop nach Anspruch 5, wobei das Antriebselement mit dem Finger bedient werden kann.

## Revendications

1. Endoscope (40, 70, 82) comprenant :
une tige allongée (41, 74) ;
un canal de travail (42, 77) s'étendant dans au moins une partie de la tige allongée ;
un orifice de charge (43) dans une partie proximale de la tige allongée pour charger un cathéter de distribution allongé (80) dans le canal de travail ; et
un pilote de dispositif (44, 78) pour mettre en contact et déplacer longitudinalement le cathéter de distribution allongé (80) chargé dans le canal de travail ;
**caractérisé en ce que** l'orifice de charge est configuré pour recevoir une cassette (81) ayant le cathéter de distribution (80) enroulé dans celui-ci et une longueur du cathéter de distribution s'étendant hors de la cassette (81) et pouvant être placée dans le canal de travail.

2. Endoscope selon la revendication 1, dans lequel le cathéter de distribution enroulé (80) est contraint par un ressort pour un déroulement et a un élément de retenue amovible pour empêcher le déroulement du cathéter de distribution enroulé.

3. Endoscope selon la revendication 2, dans lequel un élément d'entraînement est fourni, qui met en prise la longueur du cathéter de distribution s'étendant hors de la cassette et agit en poussant le cathéter dans le canal de travail.

4. Endoscope selon la revendication 3, dans lequel l'élément d'entraînement a une manivelle pour pousser le cathéter de distribution dans le canal de travail.

5. Endoscope selon la revendication 3, dans lequel l'élément d'entraînement comprend une roue à friction.

6. Endoscope selon la revendication 5, dans lequel l'élément d'entraînement peut être commandé avec les doigts.
